# EUROPEAN PATENT SPECIFICATION

(11) **EP 2 327 316 B1**
(45) Date of publication and mention of the grant of the patent: **16.11.2016**
(21) Application number: 09177423.2
(22) Date of filing: 29.11.2009
(51) Int. Cl.: A23L 33/17, A23L 33/00, A23L 33/10

(54) **METHOD OF ENHANCING MUSCLE PROTEIN SYNTHESIS**
VERFAHREN ZUR STEIGERUNG DER MUSKELPROTEINSYNTHESE
PROCÉDÉ D'AMÉLIORATION DE LA SYNTHÈSE DE PROTÉINES MUSCULAIRES

(43) Date of publication of application: 01.06.2011
(73) Proprietor: Premier Nutrition Corporation, Emeryville CA 94608 (US)
(72) Inventor: Zaltas, Eric, 1071 St-Saphorin (CH); Moore, Daniel, Ryan, 1000 Lausanne (CH); Stellingwerff, Trent, 1052 Le Mont-Sur-Lausanne (CH); Hawley, John, Alan, Melbourne, Victoria 3000 (AU); Burke, Louise, Mary, Melbourne, Victoria 3000 (AU)
(74) Representative: Brearley, Helen Rebecca

(56) References cited:
- US-A1- 2003 064 134
- US-A1- 2006 193 949
- US-A1- 2007 015 686
- US-A1- 2009 239 803
- MICHAEL SENO: 'Recommended Nutrition and Diet For a Speed and Agility Training Program', [Online] 21 August 2009, Ezine Articles Retrieved from the Internet: <URL:http://ezinearticles.com/?Recommended- Nutrition-and-Diet-For-a-Speed-and-Agility- Training-Program&id=2739424> [retrieved on 2012-02-10]
- MICHAEL SENO: 'Recommended Nutrition and Diet For a Speed and Agility Training Program', [Online] 21 August 2009, Ezine@rticles Retrieved from the Internet: <URL:http://ezinearticles.com/?expert=Micha el_Seno> [retrieved on 2015-02-11]
- MICHAEL SENO: 'Recommended Nutrition and Diet For a Speed and Agility Training Program', [Online] 10 August 2009, Ezine@rticles Retrieved from the Internet: <URL:http://ezinearticles.com/?Recommended- Nutrition-and-Diet-For-a-Speed-and-Agility- Training-Program&id=2739424> [retrieved on 2015-02-11]

## Description

### Field of the Invention

The present invention relates to a method of improving muscle protein synthesis. In particular, the invention relates to the improvement of muscle protein synthesis in an individual doing physical exercise.

### Background of the invention

Physical exercise alters the activity of proteins involved in 'turning on' protein synthesis (i.e. signalling molecules) which helps guide which muscle proteins are to be made and when. Similar to the molecular responses, the changes in protein synthesis after a single bout of exercise are largely specific to the exercise task, such as an increased synthesis of proteins involved in strength (i.e. myofibrillar) with resistance exercise and an increase of proteins involved with energy supply (i.e. mitochondrial) with aerobic exercise (Coffey VG, and Hawley JA. The molecular bases of training adaptation. J Sports Sci 2007). However, beyond pure endurance/aerobic exercise and pure resistance exercise, current state of the art dictates that there is no idea what proteins will be synthesized after a bout of repeated sprints exercise, similar to what team sport athletes undertake in training and competition.

For physical exercise overtime, these changes in signalling molecule activity and muscle protein synthesis summate over a period of weeks to months (i.e. with training) into physiological adaptations that make an athlete better at a specific exercise task/event.

There is very little information regarding what and how nutrition can support and optimize these training adaptations. In fact the mentioned 2007 extensive review on training adaptation "The molecular bases of training adaptation" does not discuss the role that nutrition plays in supporting or enhancing the adaptation to training. Furthermore, some exercise tasks have both a resistive as well as an aerobic component (eg. sprint-type stop-and-go commonly seen in team sports) and therefore it is completely unknown what changes occur in the molecular signalling as well as the synthesis of different muscle proteins.

There are three main different types of exercise training regimes which include: 1) resistance exercise 2) anaerobic or repeated sprint type exercise and 3) endurance exercise. Each of these exercise training regimes features a divergent training response.
1) Resistance exercise is when subjects undertake explosive movements of weight, with long periods of rest, and is primarily driven by the phosphocreatine and glycolytic energy systems. This system can produce energy quickly, but fatigues quickly. The primary adaptations include increases in muscle mass (hypertrophy) by increased muscle cross-section area through repeated weight lifting training. See e.g. Hakkinen K., Neuromuscular and hormonal adaptations during strength and power training (A review. J Sports Med Phys Fitness 29: 9-26, 1989), or Hakkinen K. et. all. on Relationships between training volume, physical performance capacity, and serum hormone concentrations during prolonged training in elite weight lifters. Int J Sports Med 8 Suppl 1: 61-65, 1987.
2) Repeated sprint type training is anaerobic in nature, involves high-intensity exercise with limited recovery periods, and involves nearly purely carbohydrate metabolism with a large breakdown in muscle glycogen (glycolytic energy production). During these situations of anaerobic energy production, such as high intensity speed training, or sports involving repeated sprints, the increased load on the muscles is accomplished by an increased firing of Type IIa fibres. Finally, at very high workloads, type IIb glycolytic muscle fibres become activated to maintain the high demand of energy provision via anaerobic energy provision. However, during these situations, the high rate of anaerobic energy production exceeds the rate at which it can be oxidized aerobically within the mitochondria, and this leads to the extreme levels of lactate production found in these types of training situations (Spriet LL, Howlett RA, and Heigenhauser GJ. An enzymatic approach to lactate production in human skeletal muscle during exercise. Med Sci Sports Exerc 32: 756-763, 2000.) Recent studies have look at the adaptation to repeated sprint training and found that type IIa fibers increase, along with increases in both mitochondria and also some hypertrophy, and increases in the lactate transporters 2. (Gibala MJ, et al. Short-term sprint interval versus traditional endurance training: similar initial adaptations in human skeletal muscle and exercise performance. J Physiol 575: 901-911, 2006).
3) Endurance training is characterized by individuals doing low-intensity training over prolonged periods (e.g. > 15min). The energy system represented for endurance training includes the aerobic system, which primarily uses aerobic metabolism of fats and carbohydrates to produce the required energy within the mitochondria when ample oxygen is present. The primary adaptations include increased muscle glycogen stores and glycogen sparing at sub-maximal workloads via increased fat oxidation, enhanced lactate kinetics and morphological alterations, including greater type I fibre per muscle area, and increased capillary and mitochondrial density (Holloszy JO, and Coyle EF. Adaptations of skeletal muscle to endurance exercise and their metabolic consequences. J Appl Physiol 56: 831-838, 1984. and Holloszy JO, Rennie MJ, Hickson RC, Conlee RK, and Hagberg JM. Physiological consequences of the biochemical adaptations to endurance exercise. Ann N Y Acad Sci 301: 440-450, 1977.)

In addition, it is unknown how nutrition can enhance these adaptations after sprint-type exercise. There is a need for understanding examining the acute effects of nutrition supplementation on molecular signalling and protein synthesis after repeated sprint based exercise, such as the majority of team sport sports, like football/soccer.

Current during and post-exercise nutrition recommendations for sprint type of sports involve the ingestion of adequate carbohydrates to replenish muscle glycogen.

US 2009/0239803 relates to a bite-size carbohydrate-based product comprising a carbohydrate source comprising a glucogenic:fructogenic carbohydrate ratio ranging between about 1.5 to about 2.5 with at least 60% of the energy content of the bite-size carbohydrate-based product coming from the carbohydrate source; and sodium.

There is a need to determine the importance of other nutrients and timing of intake to enhance muscle adaptations to sprint-type/stop-and-go exercise.

The present invention seeks to address the above-described problems. The invention also aims at other objects and particularly the solution of other problems as will appear in the rest of the present description.

### Object and summary of the invention

In a first aspect, the invention provides method of enhancing muscle protein synthesis following physical exertion that comprises running repeated sprints, the method comprising administering to a human a composition 30 minutes or less prior to, during, or within 30 minutes after running repeated sprints, wherein the composition comprises:
(a) carbohydrates; and
(b) protein and/or essential amino acids;
wherein the composition has a total protein dose of 20 g to 30 g and wherein the composition has a weight ratio of carbohydrates to protein in the range of 1:1 to 3:1. Running repeated sprints may, for example, normally be found with the majority of team sports.

It has surprisingly been found that the addition of protein or essential amino acids with repeated sprint exercise has the capacity of enhancing myofibrillar protein synthesis. Myofibrillar protein is the specific protein responsible for muscle hypertrophy (growth).

Physical exercise provides a stimulus to the body that triggers a cascade of molecular signals that lead to changes in gene expression and the synthesis of proteins specific to the exercise stimulus. The physical adaptation that results from chronic training is believed to be a direct result of the accumulation of these proteins after multiple bouts of acute exercise.

A clear beneficial effect is shown on anabolic signalling and muscle protein synthesis following repeated sprint based exercise with nutrients provisions. Recommendations are to take protein and carbohydrate in close temporary timing to strenuous training sessions. There are well documented advantages of supplementation of nutrients after resistance and endurance training, but not with repeated sprints.

It has further been found that, the absence of nutrient provisions in close proximity to high-intensity sprint workout indeed is likely to impair subsequent training adaptations since the muscle protein synthesis during recovery was nearly 50% greater with nutrient provision as compared to the placebo condition which had no nutrient provision (see example details from the report

It has also been found that when nutrition can improve the adaptations to a single training session by only a small percentage over a period of weeks or months this could have a major effect on training adaptations and thus performance.

The invention also relates to a use of a composition comprising (a) carbohydrates, and (b) protein and/or essential amino acids, wherein the composition has a total protein dose of 20 g to 30 g. and wherein the composition has a weight ratio of carbohydrates to protein in the range of 1:1 to 3:1, for improving muscle protein synthesis wherein the use is in connection with repeated sprint exercise.

Furthermore, as described above, repeated sprint type training is anaerobic in nature, and involves nearly purely carbohydrate metabolism with a large breakdown in muscle glycogen. Accordingly, nutritional recommendations are for at least 1 to 1.5g of carbohydrate per kg body mass (a total of ~50 to 75g carbohydrate) to be consumed in the first several hours after this type of exercise training.

### Brief description of the drawings

Further features, advantages and objects of the present invention will become apparent for the skilled person when reading the following detailed description of embodiments of the present invention, when taking in conjunction with the figures of the enclosed drawings.
Figure 1 shows in myofibrillar protein synthetic rate resulted from repeated sprint exercise.
Figure 2 shows plasma essential amino acid (EAA, panel A), branched-chain amino acid (BCAA, panel B) and total amino acid (panel C) concentration at rest and following repeated sprint cycling repetitions after prior ingestion of placebo or nutrient beverage 30 min before exercise.
Figure 3. p70 s6K thr389 (panel A) phosphorylation at rest and following maximal effort repeated sprint cycling repetitions after prior ingestion of placebo or nutrient beverage 30 min before exercise.

### Detailed description of embodiments

According to the invention, nutrient provision increases anabolic signalling and myofibrillar protein synthesis after repeated sprint exercise

It is commonly recommended that athletes involved in sprint-type exercise consume adequate carbohydrate to enhance glycogen resynthesis and energy recovery. Our data demonstrate for the first time the importance of protein ingestion in close proximity to a bout of sprint-type exercise in order to maximize myofibrillar protein synthesis, which does not occur with carbohydrate ingestion alone. In fact we found a 48% increase in myofibrillar protein synthesis after protein+leucine intake as compared to isocaloric carbohydrate only trial. In addition, proteins involved in 'turning on' protein synthesis are also enhanced with protein ingestion after exercise demonstrating a clear link between the signals to activate protein synthesis and the subsequent muscle protein synthetic response (e.g. ~300% increase in p70S6kinase and a ~100% increase in 4E-BP1). The activation of p70S6kinase has been shown to be important for the acute increase in muscle protein synthesis and the chronic increase in muscle growth with training. Therefore, overtime this continual timed ingestion of protein with sprint-type exercise will lead to greater muscle growth and muscle power with training; this is a very favourable adaptation for athletes engaged in this type of exercise/sport.

In order to maximize muscle protein synthesis and enhance adaptations to sprint-type exercise (eg. team sport stop-and-go) it is recommended that athletes consume a product that contains 20-35, preferably from 20 to 30 g of protein with added leucine (up to 5g) in close proximity to (i.e. 30-60min before and/or 0-120 advantageously, within 0-30 min of the exercise). These nutritional guidelines would apply to all stop-and-go individual and team sports that are characterized by periods of high intensity sprints interspersed by periods of low-intensity recovery (eg. football, tennis, ice hockey, basketball, etc.).

The composition according to the invention comprises carbohydrates and protein or essential amino acids in a carbohydrate to protein ratio in the range from 1:1 to 3:1, preferably in a ratio of 2:1.

Advantageously to maximize post-exercise glycogen resynthesis, for carbohydrate intake, the recommendation would be 1 to 1.5g CHO/kg.

The composition according to the invention comprises a total protein dose of 20 to 30 grams protein.

In an embodiment, the protein or amino acid constitute about 20% to about 40% by weight of the solids in the final product. In another embodiment, the protein or amino acid comprises about 30% by weight of the product.

Moreover, the composition can be made so that there is a consistent and countable quantity of protein per single dose, for example, between about 2 grams to about 4 grams per dose.

Preferably, the composition comprises a protein or essential amino acid content of about 2 grams to about 2.5 grams.

The composition may be in the form of a solid product, a gel, a liquid, or a ready to mix powder.

The protein-based product can also contain a discrete amount of fat in one or more products to provide any suitable amount of energy to an athlete. For example, each of the products can provide a fat amount up to 9g/300 cal. In another example the products can provide 11g/360cal. Each of the products can also provide a saturated fat amount up to 4g/300 cal or more. In an embodiment, the percentage of energy (e.g. in the form of calories) coming from fat can be up to about 25%.

In an embodiment, the protein-based product comprises an amount of fat ranging from about 10% to about 40% by weight of the protein-based product. Preferably, the protein-based product comprises an amount of fat of about 30% by weight of the protein-based product.

Any suitable individual protein, amino acid or blends of proteins can be used. For example, the protein-based product can comprise a protein blend comprising soy protein isolates, whey protein isolates and calcium caseinate. An example of the protein blend is the Tri-source^{™} protein blend.

The presence of proteins and/or fats in the nutritional product of the present disclosure has the advantage in that it is possible to provide an athlete with a more complete nutrition during performance. For the protein source, any suitable dietary protein may be such as, for example, animal proteins (e.g. milk proteins, meat proteins and egg proteins); vegetable proteins (e.g. soy protein, wheat protein, rice protein, and pea protein); mixtures of free amino acids; or combinations thereof. Milk proteins, such as casein and whey milk proteins, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example, for athletes believed to be at risk of developing cows' milk allergy. Generally, at least partially hydrolysed proteins are easier and faster to metabolize by the body. This is in particular true for amino acids. In an embodiment, the protein-based product contains single/essential amino acids such as, for example, leucine, valine and/or isoleucine.

In a preferred embodiment of the invention the essential amino acids comprising added Leucine. In the present context Leucine is an essential amino acid (EAA), found as part of the family of branched chain amino acids (BCAA). Ingestion of essential EAA stimulates the synthesis of skeletal muscle proteins with the branched-chain amino acids leucine, isoleucine, and valine suggested to play a critical role in this response. Of the BCAA, leucine has been investigated for its anabolic properties in many different tissues, including muscle. It is well established in cell culture and rat models that leucine increases the formation, and hence activation, of specific proteins that are involved in "turning on" protein synthesis.

Advantageously, a total does of essential amino acid dose of 5 to 25g blend of EAA that mimics the EAA in high quality proteins, preferably 10g EAA in used in a product according to the invention. It may be advantageously to use leucine in total does up to 25g.

The fat source has the advantage in providing for an improved mouth feel. Any fat source is suitable. For example, animal or plant fats may be used. To increase the nutritional value, n3-unsaturated and n6-unsaturated fatty acids may comprise the fat source. The fat source may also contain long chain fatty acids and/or medium chain fatty acids. For example, milk fat, canola oil, almond butter, peanut butter, corn oil and/or high-oleic acid sunflower oil may be used.

The repeated sprints exercise according to the invention are part of a sport, in either continual training for that sport, or in the competition itself.

### Example 1:

To investigate the effect of timed nutrition (protein+leucine ingestion) on the changes in signalling molecule activity and muscle protein synthesis after repeated sprint-type exercise, a clinical trial was performed below.

The study employed a randomised, cross-over, double blind design incorporating diet and exercise manipulation. Subjects performed the same repeated sprint exercise protocol on two separate occasions with altered nutrient intervention (protein versus placebo) with each testing session separated by a two-week recovery period.

Subjects consumed 500 mL of either a beverage containing 24 g whey protein, 4.8 g leucine, 50 g maltodextrin (nutrients) or non-caloric placebo beverage 30 min prior to commencing the exercise protocol. Time-motion analysis of several major team sport performances indicates maximal sprint activity occurs every ~50-100 s with sprint durations not exceeding 6 sec. Accordingly, in an effort to make the laboratory exercise simulation as "sports specific" as possible, subjects performed 10 x 6 s supra-maximal sprints beginning every 60 s at a load equivalent to 0.075 kp/kg body mass (BM) on an electronically-braked cycle ergometer.

Subjects rested comfortably in the supine position for 3 h after completion of the exercise bout. Muscle biopsies were taken from the vastus lateralis (thigh) to measure activation (as assessed by changes in phosphorylation) of proteins involved in "turning on" protein synthesis (i.e. regulate mRNA translation) by standard Western blotting procedures. Muscle (i.e. myofibrillar) protein synthesis was also measured by a primed constant infusion of a stable isotope amino acid (i.e. [*ring*-13C6]phenylalanine).

### Results

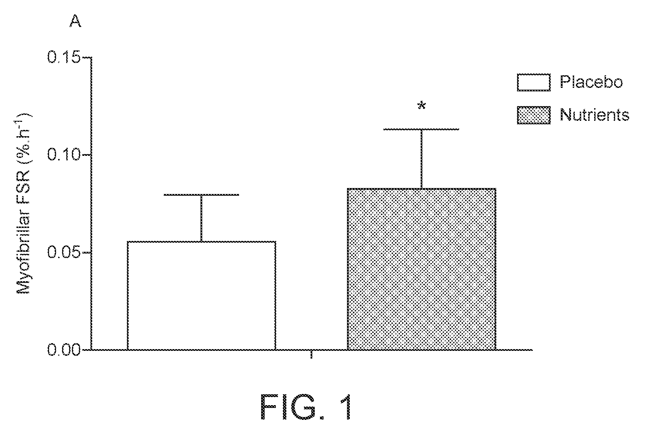

Figure 1 shows myofibrillar (panel A) protein fractional synthetic rate (FSR) during recovery (15-240 min) from 10 × 6 s maximal effort repeated sprint cycling repetitions after prior ingestion of 500 mL placebo or nutrient beverage 30 min before exercise. Values are mean ±SD.

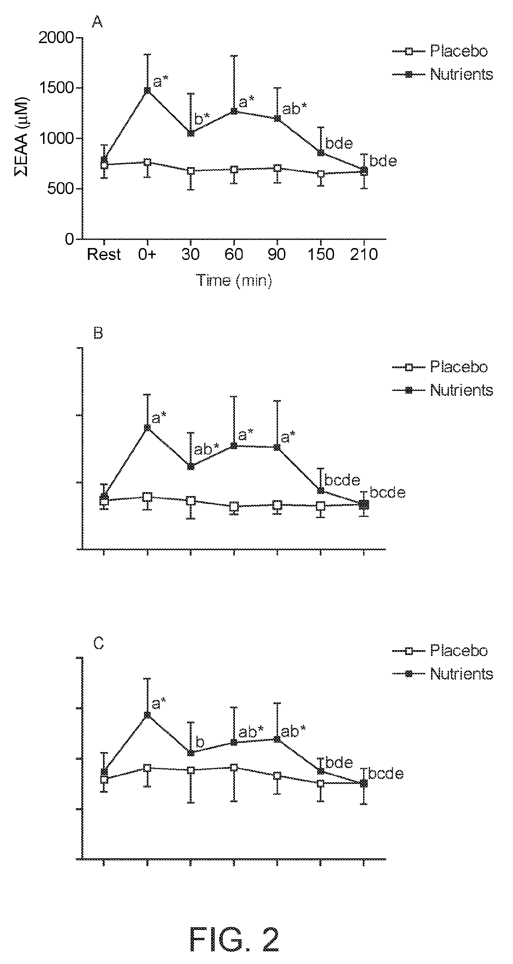

Figure 2 shows plasma essential amino acid (EAA, panel A), branched-chain amino acid (BCAA, panel B) and total amino acid (panel C) concentration at rest and following 10 × 6 s maximal effort repeated sprint cycling repetitions after prior ingestion of 500 mL placebo or nutrient beverage 30 min before exercise. Values are mean ±SD; Significantly different (P<0.05) versus (a) rest; (b) 0 min, (c) 30 min, (d) 60 min, (e) 90 min, and (*) between treatments at equivalent time-point.

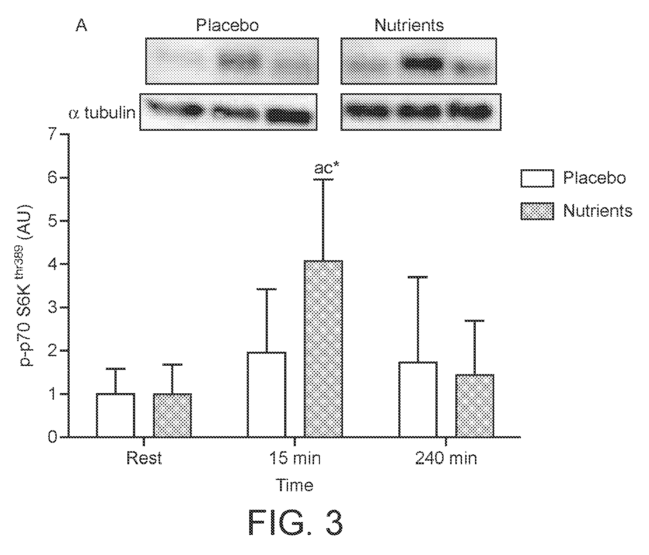

Figure 3 shows p70 S6K thr389 (panel A) phosphorylation at rest and following 10 × 6 s maximal effort repeated sprint cycling repetitions after prior ingestion of 500 mL placebo or nutrient beverage 30 min before exercise. Values are mean ±SD (n=7); Significantly different (P<0.05) versus (a) rest, (c) 240 min, and (*) between treatments at equivalent time-point.

### Blood Amino Acids (see Figure 2)

There were time × treatment interactions for plasma essential (EAA), branched-chain (BCAA) and total amino acid concentration (Figure 2). Plasma EAA concentration was only significantly elevated above rest immediately post-exercise (~85%) with nutrient provision, an effect that was also evident following 60-90 min recovery (~50-60%) (P<0.05; Figure 2A). Moreover, the increase in EAA concentration with nutrient ingestion was higher than placebo immediately post-exercise and throughout the 30-90 min recovery period (P<0.05). Likewise, plasma BCAA concentration was increased above rest immediately post-exercise and remained elevated for 90 min during recovery with nutrient but not placebo treatment (~60-120%, P<0.05; Figure 2B). The augmented post-exercise BCAA concentration with nutrient ingestion was also higher than placebo at the equivalent time-points (P<0.05).

Figure 3: There were significant time × treatment interactions for p70 S6K thr389 phosphorylation (P<0.05; Figure 4A). Phosphorylation of p70 S6K significantly increased above rest with nutrient ingestion (~300%, P<0.01) but not placebo (∼95%) 15 min after cessation of exercise resulting in a significant treatment effect (P<0.05). Despite modest elevation in p70 S6K thr389 phosphorylation following 240 min recovery these changes were not significantly different from rest (40-70%; Figure 4A).

Figure 1: Repeated sprint exercise resulted in myofibrillar protein synthetic rate that was greater with pre-exercise nutrient ingestion compared with placebo during the 15-240 min recovery period (~48%, 0.083 ±0.023 vs. 0.056 ±0.031 %·h⁻¹, P<0.05; Figure 1).

## Claims

1. A method of enhancing muscle protein synthesis following physical exertion that comprises running repeated sprints, the method comprising administering to a human a composition 30 minutes or less prior to, during, or within 30 minutes of said human running said repeated sprints, wherein the composition comprises:
(a) carbohydrates; and
(b) protein and/or essential amino acids;
wherein the composition has a total protein dose of 20 g to 30 g and wherein the composition has a weight ratio of carbohydrates to protein in the range of 1:1 to 3:1.

2. A method according to claim 1, wherein the carbohydrate to protein ratio is in the range from 1:1 to 2:1, preferably in a ratio of 2:1.

3. A method according to claims 1 or 2, wherein the essential amino acids comprises added Leucine.

4. A method according to claim 3, wherein the Leucine is at a total dose of up to 5 grams.

5. A method according to any of the preceding claims, wherein the composition comprises fat at an amount in the range of 10% to 40% by weight.

6. A method according to any of the preceding claims, wherein the protein synthesis enhanced is myofibrillar protein synthesis.

7. A method according to any of the preceding claims, wherein the composition comprises a total essential amino acid dose of 5 to 25g EAA, preferably 10 to 20g EAA.

8. A method according to any of the preceding claims, wherein the repeated sprints are part of a sport, in either continual training for that sport, or in the competition itself.

9. Use of a composition comprising (a) carbohydrates, and (b) protein and/or essential amino acids, wherein the composition has a total protein dose of 20 g to 30 g. and wherein the composition has a weight ratio of carbohydrates to protein in the range of 1:1 to 3:1, for improving muscle protein synthesis wherein the use is in connection with repeated sprint exercise.

## Patentansprüche

1. Verfahren zur Steigerung von Muskelproteinsynthese nach körperlicher Anstrengung, welche Durchführen wiederholter Sprints umfasst, wobei das Verfahren Verabreichen einer Zusammensetzung an einen Menschen 30 min oder weniger vor, während oder innerhalb von 30 min umfasst, wenn der Mensch die wiederholten Sprints durchführt, wobei die Zusammensetzung Folgendes umfasst:
(a) Kohlenhydrate; und
(b) Protein und/oder essentielle Aminosäuren;
wobei die Zusammensetzung eine Gesamtproteindosis von 20 bis 30 g aufweist und wobei die Zusammensetzung ein Gewichtsverhältnis von Kohlenhydraten zu Protein im Bereich von 1:1 bis 3:1 aufweist.

2. Verfahren nach Anspruch 1, wobei das Kohlenhydrate-Protein-Verhältnis im Bereich von 1:1 bis 2:1, bevorzugt in einem Verhältnis von 2:1, liegt.

3. Verfahren nach Anspruch 1 oder 2, wobei die essentiellen Aminosäuren hinzugefügtes Leucin umfassen.

4. Verfahren nach Anspruch 3, wobei das Leucin in einer Gesamtdosis von bis zu 5 Gramm vorhanden ist.

5. Verfahren nach einem der vorhergehenden Ansprüche, wobei die Zusammensetzung Fett in einer Menge im Bereich von 10 bis 40 Gew.-% umfasst.

6. Verfahren nach einem der vorhergehenden Ansprüche, wobei es sich bei der gesteigerten Proteinsynthese um myofibrilläre Proteinsynthese handelt.

7. Verfahren nach einem der vorhergehenden Ansprüche, wobei die Zusammensetzung eine Gesamtdosis von essentiellen Aminosäuren von 5 bis 25 g EAA, bevorzugt 10 bis 20 g EAA, umfasst.

8. Verfahren nach einem der vorhergehenden Ansprüche, wobei die wiederholten Sprints Teil eines Sports sind, entweder in kontinuierlichem Training für jenen Sport oder im Wettkampf selbst.

9. Verwendung einer Zusammensetzung, die (a) Kohlenhydrate und (b) Protein und/oder essentielle Aminosäuren umfasst, wobei die Zusammensetzung eine Gesamtproteindosis von 20 g bis 30 g aufweist und wobei die Zusammensetzung ein Gewichtsverhältnis von Kohlenhydraten zu Protein im Bereich von 1:1 bis 3:1 aufweist, zur Steigerung von Muskelproteinsynthese, wobei die Verwendung im Zusammenhang mit wiederholten Sprintübungen steht.

## Revendications

1. Procédé de stimulation de la synthèse protéique musculaire après un exercice physique qui comprend des sprints répétés, le procédé comprenant l'administration à un humain d'une composition 30 minutes ou moins avant, pendant, ou dans les 30 minutes après lesdits sprints humains répétés, dans lequel la composition comprend :
(a) des hydrates de carbone ; et
(b) une protéine et/ou des acides aminés essentiels ;
dans lequel la composition a une dose totale de protéine de 20 g à 30 g et dans lequel la composition a un rapport pondéral des hydrates de carbone à la protéine dans la plage de 1:1 à 3:1.

2. Procédé selon la revendication 1, dans lequel le rapport des hydrates de carbone à la protéine est dans la plage de 1:1 à 2:1, de préférence dans un rapport de 2:1.

3. Procédé selon les revendications 1 ou 2, dans lequel les acides aminés essentiels comprennent la leucine ajoutée.

4. Procédé selon la revendication 3, dans lequel la leucine est à une dose totale allant jusqu'à 5 grammes.

5. Procédé selon l'une quelconque des revendications précédentes, dans lequel la composition comprend de la graisse en une quantité dans la plage de 10 % à 40 % en poids.

6. Procédé selon l'une quelconque des revendications précédentes, dans lequel la synthèse protéique stimulée est la synthèse protéique myofibrillaire.

7. Procédé selon l'une quelconque des revendications précédentes, dans lequel la composition comprend une dose totale d'acides aminés essentiels de 5 à 25 g d'AAE, de préférence 10 à 20 g d'AAE.

8. Procédé selon l'une quelconque des revendications précédentes, dans lequel les sprints répétés font partie d'un sport, soit dans l'entraînement continu pour ce sport, soit dans la compétition elle-même.

9. Utilisation d'une composition comprenant (a) des hydrates de carbone, et (b) une protéine et/ou acides aminés essentiels, la composition ayant une dose totale de protéine de 20 g à 30 g, et la composition ayant un rapport pondéral des hydrates de carbone à la protéine dans la plage de 1:1 à 3:1, pour stimuler la synthèse protéique musculaire, l'utilisation étant associée à un exercice de sprint répété.
